# EUROPÄISCHE PATENTANMELDUNG

(11) **EP 1 764 132 A1**
(43) Veröffentlichungstag der Anmeldung: **21.03.2007**
(21) Anmeldenummer: 05020254.8
(22) Anmeldetag: 16.09.2005
(51) Int. Cl.: A61N 5/10

(54) **Verfahren und Vorrichtung zur Einstellung eines Strahlpfades einer Partikeltherapieanlage**

(71) Anmelder: SIEMENS AKTIENGESELLSCHAFT, 80333 München (DE)
(72) Erfinder: Birgy, Denis, 91091 Grossenseebach (DE); Breuninger, Harald, 91330 Eggolsheim (DE); Frankenbach, Peter, 65343 Eltville (DE); Rössler, Lothar, 56370 Reckenroth (DE)

(57) **Zusammenfassung**

Die Erfindung betrifft eine Partikeltherapieanlage
- mit einer Beschleuniger- und Partikelstrahlzuführungseinheit zur Beschleunigung von Partikeln und zur Zuführung von Partikeln an mindestens zwei Bestrahlungsplätze,
- mit einer Beschleunigerkontrolleinheit zur Einstellung und Kontrolle von zur Bestrahlung benötigten Parametern der Beschleuniger- und Partikelstrahlzuführungseinheit und
- mit einer Zuordnungseinheit zur Sicherstellung und Überwachung einer korrekten Partikelstrahlführung entlang eines Strahlpfades in der Beschleuniger- und Partikelstrahlzuführungseinheit zu einem den Partikelstrahl anfordernden Bestrahlungsplatz,

wobei die Beschleuniger- und Partikelstrahlzuführungseinheit mindestens ein zur Einstellung des Strahlpfades einstellbares Element aufweist, das mit der Beschleunigerkontrolleinheit zur Übermittlung mindestens eines Einstellparameters verbunden ist und das mit einem Signalausgang der Zuordnungseinheit über eine direkte und fest zugeordnete Signalverbindung zum Empfang eines Aktivierungssignals verbunden ist, wobei das Aktivierungssignal eine Umsetzung des Einstellparameters im Element veranlasst

## Beschreibung

Die Erfindung betrifft eine Partikeltherapieanlage mit einer Beschleuniger- und Partikelstrahlzuführungseinheit zur Beschleunigung von Partikeln und zur Zuführung von Partikeln an mindestens zwei Bestrahlungsplätze, mit einer Beschleunigerkontrolleinheit zur Einstellung und Kontrolle von zur Bestrahlung benötigten Parametern der Beschleuniger- und Partikelstrahlzuführungseinheit und mit einer Zuordnungseinheit zur Sicherstellung und Überwachung einer korrekten Partikelstrahlführung entlang eines Strahlpfades in der Beschleuniger- und Partikelstrahlzuführungseinheit zu einem den Partikelstrahl anfordernden Bestrahlungsplatz.

Eine Partikeltherapieanlage weist üblicherweise eine Partikelbeschleunigereinheit, eine sich anschließenden Partikelstrahlzuführungseinheit sowie mehrere Bestrahlungsplätze auf. Die Beschleunigung der Partikel, z.B. Protonen, Pionen, Helium-, Kohlenstoff- oder Sauerstoff-Ionen, erfolgt beispielsweise mit Hilfe eines Synchrotrons oder Zyklotrons. Die beschleunigten hochenergetischen Partikel werden aus der Partikelbeschleunigereinheit ausgekoppelt und in die auch als Hochenergiestrahltransportsystem (high energy beam transport system HEBT) bezeichnete Partikelstrahlzuführungseinheit eingekoppelt. Im Falle eines Synchrotrons erfolgt die Auskopplung beispielsweise über einen KO-Exciter. Die HEBT erlaubt es, die hochenergetischen Partikel immer demjenigen Bestrahlungsplatz zuzuführen, an dem gerade ein Bestrahlungsvorgang erfolgen soll.

An einem im Folgenden auch als Behandlungsplatz bezeichneten Bestrahlungsplatz erfolgt z.B. eine Tumortherapie eines Patienten, der dazu im Partikelstrahlengang positioniert und den hochenergetischen Partikel ausgesetzt wird. Man unterscheidet zwischen "fixed beam" Behandlungsplätzen, in denen die Partikel aus einer festen Richtung auf einen Behandlungsplatz treffen, und so genannten Gantry basierten Behandlungsplätzen. Bei letzteren ist es möglich, den Partikelstrahl aus verschiedenen Richtungen auf den Behandlungsplatz der Gantry zu richten. Ferner erfolgt eine Überwachung der Strahlqualität an einem im Folgenden als Überprüfungsplatz bezeichneten Bestrahlungsplatz. Dort werden Strahlparameter wie Partikelenergie, Energieverteilung und Strahlintensität in Qualitätsmessungen überwacht.

An die Sicherheit einer Partikeltherapieanlage sind hohe Ansprüche gestellt. So muss z.B. gewährleistet sein, dass der Partikelstrahl immer nur zu einem Bestrahlungsplatz geführt wird, der auf einen Bestrahlungsvorgang vorbereitet ist und der den Partikelstrahl angefordert hat. Ferner muss gewährleistet sein, dass der Partikelstrahl die korrekten angeforderten Parameter aufweist. Ferner ist im Notfall eine schnelle Unterbrechung der Partikelzuführung notwendig. Dazu weist die HEBT z.B. eine Schikane auf, die es erlaubt den Partikelstrahl schnell abzuschalten. Üblicherweise gewährleistet ein Kontroll- und Sicherheitssystem der Partikeltherapieanlage, dass jeweils ein mit den benötigten Parametern charakterisierter Partikelstrahl in den entsprechenden Behandlungsraum geführt wird.

Die benötigten Parameter werden im so genannten Behandlungs- oder Therapieplan definiert. Dieser gibt an, wie viele Teilchen aus welcher Richtung mit welcher Energie auf den Patienten treffen sollen. Die Energie der Partikel bestimmt die Eindringtiefe der Partikel in den Patienten, d.h. den Ort, an dem das Maximum der Wechselwirkung mit dem Gewebe bei der Partikeltherapie erfolgt; in anderen Worten, den Ort, an dem das Maximum der Dosis deponiert wird. Die vom Therapieplan geforderten Parameter werden üblicherweise von einer Beschleunigerkontrolleinheit in Einstellparameter, z.B. in Form von Maschinenparametern, für die Partikelbeschleunigereinheit und die Partikelstrahlzuführungseinheit umgesetzt. Ferner wird die Information, an welchen Bestrahlungsplatz der Partikelstrahl geführt werden soll, in Einstellparameter für die Partikelstrahlzuführungseinheit umgesetzt. Des Weiteren steuert eine Kontrolleinheit des Bestrahlungsplatzes beispielsweise eine Positioniervorrichtung, mit der ein zu bestrahlender Patient/ein zu bestrahlendes Phantom in Bezug zum Partikelstrahl positioniert wird.

Eine Partikeltherapieanlage mit mehreren Fixed-Beam-Behandlungsplätzen und einer Gantry ist z.B. aus EP 0 986 070 bekannt. Verschiedene Bestrahlungsanlagen und -techniken sind von H. Blattmann in "Beam delivery systems for charged particles", Radiat. Environ. Biophys. (1992) 31:219-231 beschrieben.

Ein Verfahren zur Auswahl eines Behandlungsraumes ist z.B. aus US 5,260,581 und ein Kontroll- und Sicherheitssystem für eine Strahlentherapieanlage ist z.B. aus US 5,895,926 bekannt.

Der Erfindung liegt die Aufgabe zugrunde, einen sicheren Betrieb einer Partikeltherapieanlage und insbesondere eine sichere Zuführung eines Partikelstrahls an einen den Partikelstrahl anfordernden Bestrahlungsplatz zu gewährleisten.

Diese Aufgabe wird erfindungsgemäß gelöst durch eine Partikeltherapieanlage nach Anspruch 1 sowie ein Verfahren und eine Vorrichtung zur Einstellung eines Strahlpfades nach Anspruch 13 bzw. 20. Ferner wird die Aufgabe gelöst durch die Verwendung einer direkten und fest zugeordneten Signalverbindung von einer Zuordnungseinheit einer Partikeltherapieanlage zu einem zur Einstellung eines Strahlpfades in der Partikeltherapieanlage einstellbaren Element zur Übermittlung eines Aktivierungssignals.

Eine direkte und fest zugeordnete Signalverbindung ist z.B. eine direkte Hardware-Verbindung, insbesondere eine einzelne und bevorzugt eine sicherheitsgerichtete, Signalleitung. "Dabei schließt direkt auch ein Zusammenklemmen von mehreren Kabelabschnitten ein, wobei ein einziges durchgehend verlegtes Kabel bevorzugt ist."

Eine direkte und fest zugeordnete Signalverbindung hat den Vorteil, dass eine eindeutige Zuordnung eines Elements zu einem Signalausgang besteht. Dadurch ist es gewährleistet, dass immer dieses eine Element aktiviert wird, ohne dass hier eine Bestätigung zur Verifikation der Ansteuerung des richtigen Elements über z.B. ein Protokoll benötigt wird. Es besteht eine Sicherheit bei der Einstellung des Elements ohne zusätzlichen Verifikationsschritt. Somit erlaubt eine auf diese Weise Hardware kodierte und kontrollierte Vorgehensweise ein Einstellen von Elementen z.B. einer HEBT für eine gesicherte Zuführung eines Partikelstrahls entlang eines durch die Elemente definierten Partikelpfads zum anfordernden Bestrahlungsplatz. Die Signalverbindung wird vorzugsweise nur in eine Richtung verwendet, so dass eine störanfällige Logik zur Differenzierung der Richtung der Signalübertragung nicht benötigt wird.

Das Beruhen des Sicherheitskonzepts auf einer Hardware-Kodierung ist ein Vorteil gegenüber dem Verfahren in der eingangs genannten Schrift US 5,895,926. Denn eine gesicherte Zuteilung und/oder eine gesicherte Strahlverfügbarkeitskontrolle und/oder eine gesicherte Einstellung des Strahlpfades durch dedizierte Hardware-Signalleitungen erschwert Manipulationsmöglichkeiten gegenüber einer reinen Bus-Lösung, denen eine variable Signalgebung aufprägbar ist.

Eine Ausführungsform einer erfindungsgemäßen Partikeltherapieanlage weist beispielsweise eine Beschleuniger- und Partikelstrahlzuführungseinheit zur Beschleunigung von Partikeln und zur Zuführung von Partikeln vom Beschleuniger an mindestens zwei Bestrahlungsplätze auf. Eine derartige Einheit umfasst beispielsweise als Beschleuniger ein Zyklotron oder ein Synchrotron, in das evtl. vorbeschleunigte Partikel eingekoppelt werden. Die Partikelstrahlzuführung erfolgt mit Hilfe von mindestens einem einstellbaren Element im Strahlpfad. Das oder die Elemente werden mit Hilfe der Beschleunigerkontrolleinheit entsprechend dem jeweils benötigten Strahlpfad eingestellt. Zur Einstellung benötigte Einstellparameter werden übermittelt und z.B. in einem Zwischenspeicher abgelegt.

Ferner umfasst die Partikeltherapieanlage eine Zuordnungseinheit zur Sicherstellung und Überwachung einer korrekten Partikelstrahlführung entlang des Strahlpfades. Dieser verläuft innerhalb der Beschleuniger- und Partikelstrahlzuführungseinheit zu einem den Partikelstrahl anfordernden Bestrahlungsplatz. Die Zuordnungseinheit ist beispielsweise eine sicherheitsgerichtete und speicherprogrammierbare Steuerungseinheit.

Zur Übermittlung eines Aktivierungssignals ist ein Signalausgang der Zuordnungseinheit über eine direkte und fest zugeordnete Signalverbindung mit mindestens einem der einstellbaren Elemente verbunden. Nur in Zusammenspiel mit dem Aktivierungssignal kann eine Umsetzung übermittelten Einstellparameters im Element erfolgen. D.h., das Aktivierungssignal liegt z.B. vor und/oder während der Umsetzung vor.

Prinzipiell ist es vorzugsweise so vorgesehen, dass die einstellbaren Elemente vorrangig deaktiviert sind, d.h., die Zuordnungseinheit wirkt als Verriegelungsmechanismus. Nur bei Vorliegen eines Aktivierungssignals werden entsprechende Ströme etc. eingestellt. Deaktivierung bedeutet, dass beispielsweise die Defaultwerte eingestellt sind, z.B. kein Stromfluss in den Magnetspulen.

Üblicherweise wird die Beschleuniger- und Partikelstrahlzuführungseinheit mehrere Elemente aufweisen. Diese sind jeweils einzeln über je eine fest zugeordnete Signalverbindung direkt mit je einem Signalausgang der Zuordnungseinheit verbunden. Damit sind die einstellbaren Elemente über eine direkte Hardwareverbindung beispielsweise durch einzelne, direkte Signalleitungen mit der Zuordnungseinheit verbunden.

Beispiele für einstellbare Elemente sind Strahlumlenkmagnete, die den Partikelstrahl von dem Strahlzuführungssystem in die einzelnen Behandlungsräume ablenken, eine Strahlauskopplungsvorrichtung eines Beschleunigers, beispielsweise ein Knock-Out-Exciter eines Synchrotron-Rings, ein Dipolmagnet einer Schikane im HEBT. Mögliche Einstellparameter sind entsprechend das anliegende Magnetfeld, ein dafür benötigter einzustellender Stromwert oder eine HF-Auskoppelfrequenz. Ein einstellbares Element ist vorzugsweise zur Verarbeitung und in Abhängigkeit vom Vorliegen des Aktivierungssignals zur Umsetzung des mindestens einen übermittelten Einstellparameters ausgebildet. Dazu weist es beispielsweise einen Pufferspeicher auf, in den ein übermittelter Einstellparameter abgelegt und nach Erhalt des Aktivierungssignals ausgelesen werden kann.

Eine Übermittlung der Einstellparameter erfolgt beispielsweise über ein Datenbus-System, an das die Beschleunigerkontrolleinheit und die jeweiligen Elemente angebunden sind. Der Einstellparameter wird durch den am Bestrahlungsplatz stattfindenden Bestrahlungsvorgang bedingt. Somit ist es vorteilhaft, wenn auch eine Kontrolleinheit eines der Bestrahlungsplätze zum Austausch von zur Bestrahlung benötigten Parametern des Partikelstrahls und/oder Parameter der Beschleuniger- und Partikelstrahlzuführungseinheit an dieses Datenbus-System oder an einem eigenen Datenbus-System angebunden sind.

Beispiele für Bestrahlungsplätze sind ein Behandlungsplatz zur Strahlentherapie, z.B. ein Fixed-Beam- oder Gantry-Behandlungsplatz, oder ein Überprüfungsplatz zur Überprüfung von der Partikelbestrahlung zugrunde liegenden Parametern.

Gemäß dem Verfahren zum Einstellen eines Strahlpfades werden folgende Vorgänge nacheinander oder eventuell zeitgleich ablaufen. Zum Einstellen wird mindestens ein Einstellparameter an das Element gesendet. Ferner wird ein Aktivierungssignal über eine direkte und fest zugeordnete Signalverbindung von der Zuordnungseinheit an das Element gesendet. Dieses Signal kann kurzzeitig oder über den gesamten Bestrahlungsvorgang vorliegen. Im ersten Fall bewirkt das Aktivierungssignal z.B. ein Umschalten einer Einstellung des Elements z.B. auf "einstellbar". Grundlegend ist, dass ein derartiges Aktivierungssignal während des Einstellens vorliegen oder vorgelegt haben muss um die Einstellung des Einstellparameters umsetzen zu können. Liegen der Einstellparameter und das Aktivierungssignal vor, erfolgt ein Umsetzen des Einstellparameters im Element zur gewünschten Einstellung und Wirkungsweise des Elements.

Weitere mögliche Vorgänge bei der Anforderung eines Strahls an einen Bestrahlungsplatz können folgende Schritte sein:
- Ein Anforderungssignal wird von einem der Bestrahlungsplätze an die Zuordnungseinheit insbesondere über eine direkte und feste zugeordnete Signalverbindung gesendet.
- Eine Verfügbarkeit des Partikelstrahls wird überprüft und bei einer vorliegenden Verfügbarkeit des Partikelstrahls wird der Partikelstrahl dem anfordernden Bestrahlungsplatz zugeteilt.
- Nach dem Zuteilen wird ein Bestätigungssignal an den anfordernden Bestrahlungsplatz, beispielsweise ebenfalls über eine direkte und feste zugeordnete Signalverbindung oder über ein Datenbussystem, geschickt.
- Ferner kann bei Eintreten eines Fehlers die Partikelbeschleunigung und/oder einer Weiterleitung der Partikel beispielsweise von der Zuordnungseinheit und/oder der Kontrolleinheit unterbrochen werden. Dazu wird, falls beispielsweise für ein Einstellen des Elements fortwährend ein Aktivierungssignal vorliegen muss, dieses Aktivierungssignal beendet. Wirkt das Aktivierungssignal als Schalter, kann des Element auf "nicht-einstellbar" geschaltet werden.

Weitere vorteilhafte Ausführungsformen der Erfindung sind durch die Merkmale der Unteransprüche gekennzeichnet.

Es folgt die Erläuterung von mehreren Gesichtspunkten der Erfindung anhand der Figuren 1 bis 3. Es zeigen:
Figur 1 einen schematischen Überblick über eine Therapieanlage,
Figur 2 eine schematische sicherheitsgerichtete Schalteinheit und
Figur 3 eine Skizze zur Anordnung von sicherheitsgerichteten Verbindungen.

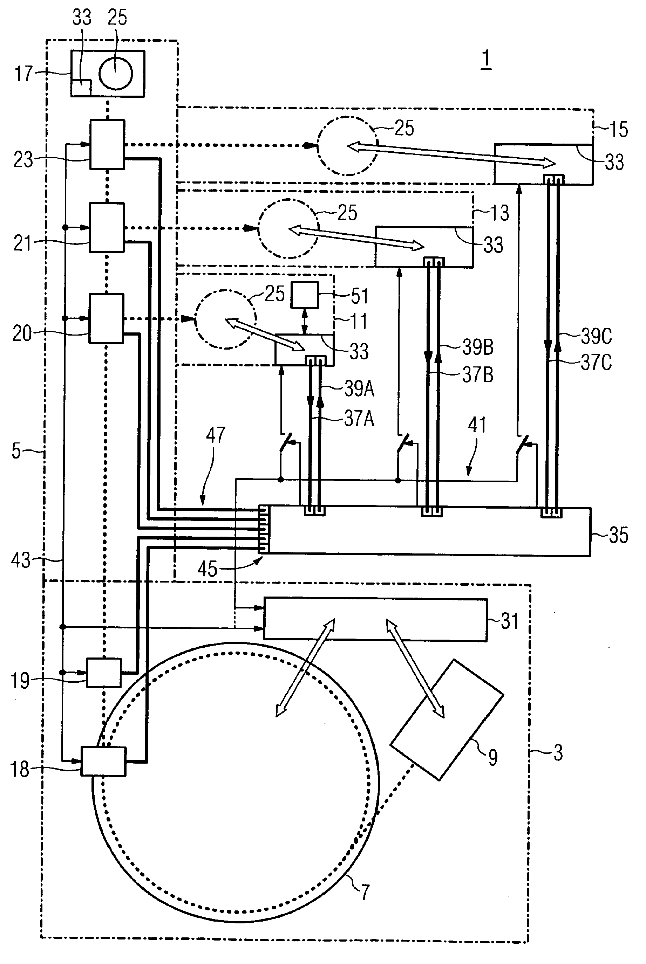

Figur 1 zeigt schematisch eine Therapieanlage 1 und verdeutlicht das Zusammenspiel von verschiedenen involvierten Kontrolleinheiten. Diese bewirken und überwachen die Einstellung von Komponenten, um einen Strahl mit entsprechenden Parametern an einen Bestrahlungsplatz zu senden. Aus Sicherheitsgründen werden dabei wichtige Signale über Fehler unanfällige Hardware-Verbindung übermittelt. Die Hardware-Verbindung besteht z.B. aus einer eigenen, separaten spezifischen Leitung und ist eindeutig der Übertragung eines Signals zugeordnet.

Die Therapieanlage 1 weist eine Beschleunigereinheit 3 und eine Partikelstrahlzuführungseinheit 5 auf. Beispielhaft für einen Beschleuniger wurde ein Synchrotron 7 mit einer vorgeschaltenen Linearbeschleunigereinheit 9 dargestellt. Die Strahlzuführungseinheit 5 verteilt die Partikel auf mehrere Bestrahlungsplätze. Beispielhaft sind schematisch drei Behandlungsplätze 11, 13 und 15 zur Strahlentherapie und ein Überprüfungsplatz 17 zur Gewährleistung der Qualität des Partikelstrahls angedeutet. Am Überprüfungsplatz 17 erfolgt beispielsweise die Qualitätssicherung mit Hilfe von Qualitätsprozeduren, beispielsweise mit regelmäßigen Tests zur Verifikation der zuvor definierten Strahlparameter, beispielsweise von Positions- und Intensitätsabstufungen, von Partikelenergien. Letztere sind beispielsweise in einer Bibliothek enthalten und werden durch automatisierte Bragg-Peak-Messungen an Phantomen überprüft.

Mithilfe einer Auskoppelvorrichtung 18 werden im beispielsweise Synchrotronring 7 gespeicherte Partikel ausgekoppelt und in die Strahlzuführungseinheit 5 eingekoppelt. Die Möglichkeit einer schnellen Strahlabschaltung nach Beendigung oder Unterbrechung des Bestrahlungsvorgangs erfolgt beispielsweise durch den Einbau einer Schikane 19, welche beispielsweise aus drei kleinen Dipol-Magneten besteht, die nach der Extraktionseinheit 18 angeordnet sind. Durch eine schnelle Abschaltung des beispielsweise mittleren Dipols wird der Strahl an einem Kollimator vernichtet.

Die Zuführung der Partikel zu den Bestrahlungsplätzen 11, 13 und 15 erfolgt durch die Ablenkung des Partikelstrahls mittels Umlenkmagnete 20, 21 und 23 aus einer Hauptstrahlrichtung in der Strahlzuführungseinheit 5. In Hauptstrahlrichtung befindet sich der Überprüfungsplatz 17. An den Bestrahlungsplätzen erfolgt die Wechselwirkung der Partikel mit einem zu bestrahlenden Patienten oder einem Phantom in Bestrahlungszonen 25. Eine der Bestrahlungszonen 25 ist z.B. durch einen maximal abscanbaren Scanbereich einer (Raster- )Scanvorrichtung, einen maximal bestrahlbaren Scatterbereich einer Scattervorrichtung oder einen einstellbaren Gantry-Bestrahlungsbereich etc. gegeben.

Die Linearbeschleunigereinheit 9 kann beispielsweise ein oder mehrere betreibbare Ionenquellen, eine Niedrigenergiestrahlführung, einen Radio-Frequenz-Quadrupol, einen Drift-Röhrenbeschleuniger und eine Injektionsstrahlführung aufweisen. Aufgaben der Injektoreinheit 9 sind die Erzeugung einer oder mehrerer Teilchensorten, deren Befreiung von Verunreinigungen durch nicht gewünschte Teilchensorten, die Einstellung der Strahlintensität im Niedrigenergiebereich beispielsweise für das Synchrotron, eine Vorbeschleunigung der Partikel und eine Vorbereitung des Partikelstrahls beispielsweise in der Pulslänge und den Strahlparametern entsprechend den Anforderungen des Synchrotrons.

Wird die Therapieanlage 1 zur Bestrahlung mittels Scanning-Verfahren eingesetzt, ist eine langsame Extraktion von Vorteil, die eine optimale Nutzung der beschleunigten Teilchen und eine präzise Strahlüberwachung während der Tumorabtastung ermöglicht. In diesem Fall ist eine Strahlextraktion mit einer sog. HF-Knock-Out-Methode von Vorteil, bei der ein Knock-Out-Exciter die Auskoppeleinheit 18 bildet.

Das Kontroll- und Sicherheitssystem der Therapieanlage 1 ist zur Verdeutlichung in mehrere Komponenten aufgeteilt. Eine Aufteilung kann auch anders oder gar nicht vorgenommen werden, solange die verschiedenen Aspekte bei der Überwachung berücksichtigt werden.

In der Ausführung nach Figur 1 sorgt eine Beschleunigerkontrolleinheit 31 dafür, dass der angeforderte Partikelstrahl entsprechend seiner Spezifikation im Behandlungsraum ankommt. An den Bestrahlungsplätzen angeordnete Kontrolleinheiten 33 steuern den Ablauf eines Bestrahlungsvorgangs und sorgen dafür, dass der Partikelstrahl entsprechend einer Bestrahlungsplanung auf einen Patienten trifft.

Ferner umfasst das Kontroll- und Sicherheitssystem eine Zuordnungseinheit 35. Diese gewährleistet es, dass nur derjenige Bestrahlungsplatz 11,...17 einen Partikelstrahl zugeführt bekommt, der ihn auch angefordert hat. Dazu ist die Zuordnungseinheit 35 einerseits mit den Kontrolleinheiten 33 zumindest zur Übermittlung eines Anforderungssignals über eine feste und eindeutig zugeordnete Signalleitung 37A, 37B, 37C verbunden. Ferner kann eine weitere fest zugeordnete Signalleitung 39A, 39B, 39C zwischen der Zuordnungseinheit 35 und den Kontrolleinheiten 33 bestehen. Diese kann beispielsweise zur Übermittlung eines Bestätigungssignals von der Zuordnungseinheit 35 zu demjenigen Bestrahlungsplatz, dem als nächstes der Partikelstrahl zugeführt werden soll, verwendet werden.

Vorzugsweise weist das Kontroll- und Sicherheitssystem mindestens ein Datenbus-System 41 auf, an das die Kontrolleinheiten 33 und die Beschleunigerkontrolleinheit 31 angebunden sind. Es dient beispielsweise der Übermittlung von Einstellparametern für die Beschleunigereinheit 3 und die Partikelstrahlzuführungseinheit 5 für eine nächste durchzuführende Bestrahlung. Vorzugsweise kann die Zuordnungseinheit 35 derart auf das Datenbus-System 41 einwirken, dass nur derjenige Bestrahlungsplatz 11,...17, der ein Bestätigungssignal bekommen hat, Parameter übermitteln kann.

An ein weiteres, eventuell auch mit dem Datenbusses 41 zusammengelegtes, Datenbussystem 43 (gestrichelte Verbindung), sind die Beschleunigerkontrolleinheit 31 und von diesem einstellbare Elementen der Beschleuniger- und Strahlzuführungseinheit angebunden. In der Ausführungsform gemäß Figur 1 sind dies beispielsweise die Auskoppeleinheit 18, die Schikane 19 und die Umlenkmagneten 20, 21, 23. Über das Datenbussystem 43 werden diejenigen Einstellparameter an die Elemente übermittelt, die diese Elemente zur Einstellung des aktuell angeforderten Partikelstrahlpfades und für den Transport der Partikel mit der richtigen Energie etc. benötigen. Ein Strahlpfad bedingt Einstellungen von Elemente im Hochenergiestrahlpfad in Abhängigkeit eines festgelegten Bestrahlungsplatzes.

Allerdings kann eine Umsetzung der Einstellparamater nur dann erfolgen, wenn zusätzlich ein Aktivierungssignal der Zuordnungseinheit 35 an dem jeweils einzustellenden Element vorliegt. Dazu sind die einstellbaren Elemente mit Signalausgängen 45 der Signalzuordnungseinheit 35 über direkte, fest zugeordnete Signalleitungen 47 verbunden.

Die Tatsache, dass Anforderungs- und/oder Aktivierungssignale über spezifische unzweideutige Hardware-Verbindungen gesendet und empfangen werden, reicht aus zu gewährleisten, dass das Anforderungssignal von einem gewissen und bekannten Bestrahlungsplatz gesendet wurde und/oder dass nur explizit aktivierte Elemente zur Festlegung des Strahlpfades eingestellt werden. Es ist nicht möglich, dass Signale fehlerhaft von anderen Bestrahlungsplätzen empfangen oder zu anderen Elementen übermittelt werden.

Im Folgenden wird ein sicherer Ablauf einer Bestrahlung eines Patienten beschrieben, wie er beispielsweise unter Verwendung einer Anlage nach Figur 1 erfolgen kann. In einem Therapieplan 51 wird die Bestrahlung mit all seinen erforderlichen Parametern wie Strahleinfallsrichtung, Strahlintensität, Partikelart, Partikelenergie etc. festgelegt.

Nachdem der Bestrahlungsplan für den Patienten am Bestrahlungsplatz geladen, alle sicherheitstechnischen Voraussetzungen erfüllt und der Patient entsprechend positioniert wurde, fordert ein Therapiekontrollsystem beispielsweise eine Kontrolleinheit der Bestrahlungsplätze 11,... 15 einen Strahl mit den geplanten Parametern für den aktuellen Bestrahlungsplatz an. Vorzugsweise können nur getestete und freigegebene Datensätze von Parametern verwendet und angefordert werden, welche im Beschleunigerkontrollsystem 31 abgespeichert vorliegen.

Für die Strahlanforderung veranlasst ein Bediener die Sendung eines Anforderungssignals von beispielsweise der Kontrolleinheit des Bestrahlungsplatzes 11 entlang der direkten fest zugeordneten Signalleitung 37A an die Zuordnungseinheit 35. Die Zuordnungseinheit 35 überprüft die Verfügbarkeit des Partikelstrahls. Findet noch an einem Nachbarbestrahlungsplatz ein Bestrahlungsvorgang statt, teilt die Zuordnungseinheit erst mit Beendigung dieses Bestrahlungsvorgangs dem anfordernden Behandlungsraum den Partikelstrahl zu. Die Zuordnungseinheit gibt beispielsweise erst zu diesem Zeitpunkt die Verbindung von der Kontrolleinheit 33 des Behandlungsraums 11 zur Beschleunigerkontrolleinheit 31 im Datenbus-System 41 für die Übermittlung der gewünschten Parameter für den folgenden Bestrahlungsvorgang frei.

Zusätzlich sendet die Zuordnungseinheit 35 an die für die Strahlzuführung zum anfordernden Bestrahlungsplatz benötigten einstellbaren Elemente, in diesem Fall die Auskoppeleinheit 18, die Schikane 19 und der Umlenkmagnet 20, über die fest zugeordneten Signalleitungen 47 Aktivierungssignale. Ferner übermittelt die Beschleunigerkontrolleinheit 31 Einstellparameter in diese Elementen. Nur bei Vorlage des Aktivierungssignals können die von der Beschleunigerkontrolleinheit 31 übermittelten Einstellparameter in den Elementen umgesetzt werden und den benötigten Partikelstrahlpfad festlegen. Vorzugsweise sind die einstellbaren Elemente vorrangig deaktiviert. Nur bei Vorliegen eines Aktivierungssignals werden entsprechende Ströme etc. eingestellt. Deaktivierung bedeutet, dass beispielsweise der Defaultwert "Strom auf Null" eingestellt wird.

Nach erfolgter Einstellung übermittelt die Zuordnungseinheit 35 entlang der Verbindungsleitung 39A ein Bestätigungssignal. Nach einer evtl. Bestätigung dieses Signals durch den Behandlungsplatz 11 erfolgt die Zuführung von Partikeln zur Bestrahlung im Bestrahlungsbereich 25.

Die Reihenfolge von Einstellvorgängen und Signalübermittlungen ist -bis auf das Vorhandensein eines Aktivierungssignals für die tatsächliche Umsetzung von physikalischen Einstellungen- frei gestaltbar. So kann bei einem alternativen Ablauf beispielsweise direkt nach der Zuordnung des Partikelstrahls an den Behandlungsraum 11 das Bestätigungssignal an den Behandlungsraum 11 entlang der Verbindungsleitung 39A übermittelt werden. Ein aktiv von Seiten der Kontrolleinheit 33 des Behandlungsraums auf das Bestätigungssignal ausgelöstes "Strahl an"-Signal veranlasst Aktivierungssignale von der Zuordnungseinheit sowie die Übermittlung von Einstellparameter von der Beschleunigerkontrolleinheit 31 an die relevanten Elemente. Anschließend erfolgt die physikalische Umsetzung der Einstellparameter in den Elementen und die Partikel werden dem Bestrahlungsplatz zugeführt. Dieser Ablauf hat den Vorteil, dass die Umsetzung erst nach erfolgtem Bestätigungssignal vorgenommen wird und eine eventuelle Fehleinstellung somit frühzeitig verhindert werden kann.
Beispielsweise kann im Fall, dass eine nicht anfordernde Kontrolleinheit ein Bestätigungssignal erhält, automatisch eine entsprechende Deaktivierung vorgenommen werden.

Das zuletzt beschriebene Vorgehen lässt sich in drei Stufen untergliedern. In einer ersten Stufe zur Vorbereitung kommunizieren nur die Kontrolleinheit und die Zuordnungseinheit (Strahlanfragesignal, Bestätigungssignal der Strahlzuordnung sowie "Strahl an"-Signal. In einer zweiten Stufe der Einstellung kommunizieren die zugeordnete Kontrolleinheit und die Beschleunigerkontrolleinheit, d.h., es werden entsprechende Strahlparameter angefordert und die entsprechenden Parameter an die Elemente und die Beschleunigereinheit übermittelt. In einer dritten Stufe der Aktivierung kommuniziert die Zuordnungseinheit direkt mit den Elementen und macht die jeweils benötigten Elemente einstellbar, so dass die von der Beschleunigerkontrolleinheit übermittelten Parameter umgesetzt werden können. Die dritte Stufe macht die Einstellung physikalisch möglich und setzt sie um; sie kann auch schon zeitgleich mit der zweiten Stufe erfolgen.
Während des Bestrahlungsvorgangs arbeitet die Therapieanlage vollkommen autark. Z.B. steuert die Kontrolleinheit 33 Scannermagnete und Strahldiagnoseeinheiten zur Überwachung der Strahlqualität. Die einzige Eingriffsmöglichkeit für das Betriebspersonal ist ein Abbruch des Bestrahlungsvorgangs. Im Fall eines ausgelösten Strahlabbruchs oder eines anderweitig erkannten Fehlers im System entzieht die Zuordnungseinheit 35 über die direkten und fest zugeordneten Signalleitungen zu den einstellbaren Elementen die Erlaubnis, aktiviert zu sein. Bei Eintritt eines derartigen Fehlers erfolgt z.B. eine Strahlvernichtung innerhalb der Schikane durch Herunterfahren eines Dipolmagnetfeldes. Zusätzlich werden z.B. die Umlenkmagnete 20,21,23 stromlos geschaltet und der KO-Exciter ausgeschaltet.

Nach Abschluss eines Bestrahlungsvorganges können die einstellbaren Elemente wieder auf ihre Default-Werte eingestellt; so werden z.B. die Umlenk- und/oder Schikanenmagnetfelder auf Null gefahren sowie die KO-Frequenz ausgeschaltet. In Wechselwirkung mit einem die Ausnutzung optimierenden Betriebssystems der Therapieanlage zur Steuerung von anfallenden Bestrahlungsvorgängen kann ein Default-Wert evtl. in Hinblick auf den als nächstes erfolgenden Bestrahlungsvorgangs durch entsprechende Steuerung der Zuordnungseinheit übersprungen werden, so dass der Strahlenpfad schneller für den folgenden Bestrahlungsvorgang zur Verfügung steht.

Die Aufgaben der verschiedenen Komponenten des Kontroll- und Sicherheitssystems für die Strahlanforderung und Strahlpfadfestlegung lassen sich wie folgt zusammenfassen:
- Die Beschleunigerkontrolleinheit 31 kontrolliert die richtigen Werte der Einstellparameter für die einstellbaren Elemente in der Beschleuniger- und Strahlzuführungseinheit.
- Die Zuordnungseinheit 35 gewährt die Einstellbarkeit dieser Parameter mittels eines Aktivierungsprozesses, bei dem gezielt nur diejenigen Elemente aktiviert werden, die für einen Strahlpfad notwendig sind. Die Zuordnungseinheit hat hierfür vorzugsweise eine Tabelle mit den möglichen Strahlpfaden in beispielsweise einem Lock-up-Table gespeichert. Zusätzlich erfolgt eine Prüfung der Verfügbarkeit des Partikelstrahls innerhalb der bevorzugt als sicherheitsgerichtete speicherprogrammierbare Steuerung ausgebildeten Zuordnungseinheit 35. Eine Zuteilung des Partikelstrahls erfolgt nur bei dessen Verfügbarkeit.
- Die Kontrolleinheiten in den Bestrahlungsplätzen liefern die Daten aus dem Bestrahlungsplan und entscheiden letztendlich über die Zuführung des Strahls, d.h., sie lösen die Strahlzuführung an dem entsprechenden Bestrahlungsplatz aus.

Die Verwendung der direkten und fest zugeordneten Verbindungen ist nicht auf die in der Figur skizzierte Ausführungsform beschränkt. So kann beispielsweise nur die Verbindung zwischen einem der einstellbaren Elementen und der Zuordnungseinheit derart ausgeführt sein.

Diese Aufteilung ist beispielhaft zu sehen. Z.B. können Funktionen auch innerhalb einer Einheit zusammengefasst werden, beispielsweise können Parameter für den Therapieplan direkt und nicht über eine Kontrolleinheit eines Behandlungsraumes der Beschleunigerkontrolleinheit zugeführt werden.

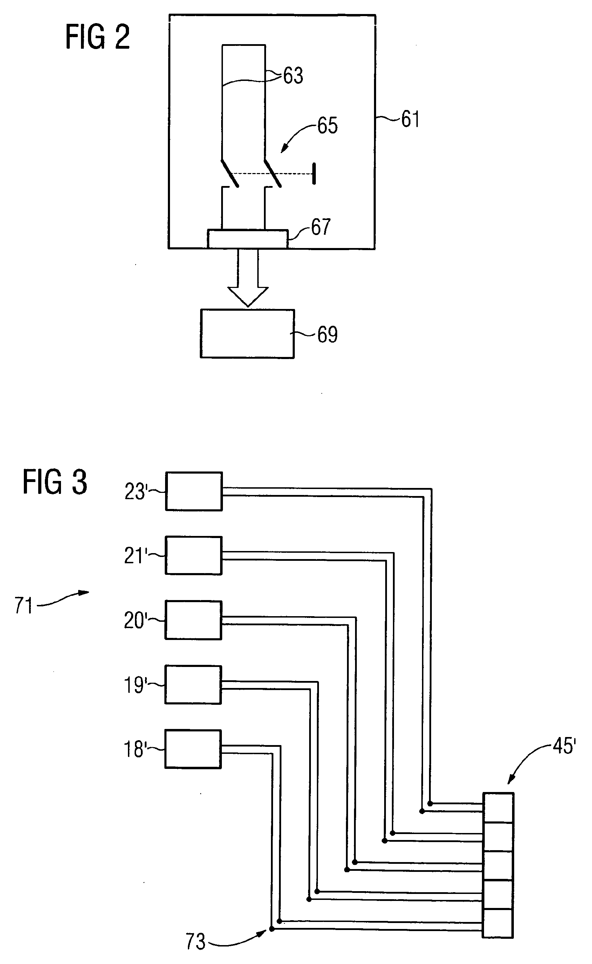

In den Figuren 2 und 3 werden Aspekte einer sicherheitsgerichteten direkten Verbindung verdeutlicht. Figur 2 zeigt schematisch eine sicherheitsgerichtete Schalteinheit 61, wie sie z.B. in der Kontrolleinheit und/oder der Zuordnungseinheit z.B. zur Übermittlung eines Anforderungs-, Bestätigungs- und/oder Aktivierungssignals verwendet werden kann. Zwei parallel geschaltete Leitungen 63 sind über einen zwangsöffnenden/zwangsschließenden Schalter 65 mit einem Signalausgang 67 verbindbar. Der Schalter 65 öffnet/schließt die beiden Leitungen 63 gemeinsam und nimmt im Fehlerfall einen sicheren Zustand ein. Der Signalausgang ist mit einer Einheit 69 verbunden; d.h. übertragen auf Figur 1 ist die Einheit 69 z.B. eine der Kontrolleinheiten 33, die Zuordnungseinheit 35 oder eines der aktivierbaren Elemente wie Auskoppeleinheit 18, Schikane 19 oder einer der Umlenkmagnete 20, 21, 23.

Figur 3 verdeutlicht den Einsatz von sicherheitsgerichteten Doppel-Leitungen für die Übermittlung von Aktivierungssignalen an einzustellenden Elemente 71 wie eine Auskoppeleinheit 18', eine Schikane 19' und Umlenkmagnete 20', 21', 23'. Diese werden von entsprechenden sicherheitsgerichtete Schalteinheit an Signalausgängen 45' angelegt, welche gemäß Figur 1 Teil der Zuordnungseinheit sind. Evtl. ist der Einsatz von Aufklemmungen 73 aufgrund der Größe einer Therapieanlage nicht vermeidbar.

## Patentansprüche

1. Partikeltherapieanlage
- mit einer Beschleuniger- und Partikelstrahlzuführungseinheit zur Beschleunigung von Partikeln und zur Zuführung von Partikeln an mindestens zwei Bestrahlungsplätze,
- mit einer Beschleunigerkontrolleinheit zur Einstellung und Kontrolle von zur Bestrahlung benötigten Parametern der Beschleuniger- und Partikelstrahlzuführungseinheit und
- mit einer Zuordnungseinheit zur Sicherstellung und Überwachung einer korrekten Partikelstrahlführung entlang eines Strahlpfades in der Beschleuniger- und Partikelstrahlzuführungseinheit zu einem den Partikelstrahl anfordernden Bestrahlungsplatz,
wobei die Beschleuniger- und Partikelstrahlzuführungseinheit mindestens ein zur Einstellung des Strahlpfades einstellbares Element aufweist, das mit der Beschleunigerkontrolleinheit zur Übermittlung mindestens eines Einstellparameters verbunden ist und das mit einem Signalausgang der Zuordnungseinheit über eine direkte und fest zugeordnete Signalverbindung zum Empfang mindestens eines Aktivierungssignals verbunden ist, wobei das Aktivierungssignal eine Umsetzung des Einstellparameters im Element veranlasst.

2. Partikeltherapieanlage nach Anspruch 1, wobei die Signalverbindungen eine direkte Hardware-Verbindung, insbesondere eine einzelne Signalleitung, ist.

3. Partikeltherapieanlage nach Anspruch 1 oder 2, wobei die Beschleuniger- und Partikelstrahlzuführungseinheit mehrere Elemente aufweist, die einzeln über je eine fest zugeordnete Signalverbindung direkt, insbesondere Hardware kodiert, mit je mindestens einem Signalausgang der Zuordnungseinheit verbunden sind.

4. Partikeltherapieanlage nach einem der Ansprüche 1 bis 3, wobei das mindestens eine Element ein Strahlumlenkmagnet, eine Strahlauskopplungsvorrichtung eines Beschleunigers, insbesondere ein KO-Exciter eines Synchrotron-Rings, oder ein der Strahlauskopplungsvorrichtung nachfolgender Umlenkmagnet ist.

5. Partikeltherapieanlage nach einem der Ansprüche 1 bis 4, wobei der mindestens eine Einstellparameter ein Wert für ein Magnetfeld, ein Stromwert oder eine Auskoppelfrequenz ist.

6. Partikeltherapieanlage nach einem der Ansprüche 1 bis 5, wobei das Element zur Verarbeitung und in Abhängigkeit vom Vorliegen des mindestens einen Aktivierungssignals zur Umsetzung des mindestens einen übermittelten Einstellparameters ausgebildet ist.

7. Partikeltherapieanlage nach einem der Ansprüche 1 bis 6, wobei das Element einen Speicher zum Abspeichern des mindestens einen Einstellparameters aufweist, aus dem nach Erhalt des Aktivierungssignals der Einstellparameter zur Umsetzung auslesbar ist.

8. Partikeltherapieanlage nach einem der Ansprüche 1 bis 7, wobei die Beschleunigerkontrolleinheit und das mindestens eine Element zur Übermittlung des mindestens einen Einstellparameters an einem ersten Datenbus-System angebunden sind.

9. Partikeltherapieanlage nach einem der Ansprüche 1 bis 8, wobei eine Beschleunigerkontrolleinheit und mindestens eine Kontrolleinheit eines der Bestrahlungsplätze zum Austausch von zur Bestrahlung benötigten Parametern des Partikelstrahls und/oder der Beschleuniger- und Partikelstrahlzuführungseinheit, welche insbesondere aufgrund eines Therapieplans festgelegt sind, an dem ersten oder an einem zweiten Datenbus-System angebunden sind.

10. Partikeltherapieanlage nach einem der Ansprüche 1 bis 9, wobei mindestens einer der Bestrahlungsplätze ein Behandlungsplatz ist, an dem ein Patient mit den Partikeln bestrahlt wird.

11. Partikeltherapieanlage nach einem der Ansprüche 1 bis 10, wobei mindestens einer der Bestrahlungsplätze ein Überprüfungsplatz zur Überprüfung von eine Partikelbestrahlung charakterisierenden Parametern ist.

12. Partikeltherapieanlage nach einem der Ansprüche 1 bis 11, wobei die Zuordnungseinheit als sicherheitsgerichtete speicherprogrammierbare Steuerungseinheit ausgebildet ist.

13. Verfahren zum Einstellen eines Strahlpfades zu einem von mindestens zwei Bestrahlungsplätzen einer Partikeltherapieanlage, wobei der Strahlpfad in einer Beschleuniger- und Partikelstrahlzuführungseinheit der Partikeltherapieanlage mittels mindestens einem Element einstellbar ist und wobei eine korrekte Einstellung des Strahlpfades mittels einer Zuordnungseinheit gewährleistet wird, mit folgenden Verfahrensmerkmalen:
- Senden von mindestens einem Einstellparametern an das mindestens eine Element,
- Senden mindestens eines Aktivierungssignals über eine direkte und fest zugeordnete Signalverbindung von der Zuordnungseinheit an das mindestens eine Element,
- Umsetzen des mindestens einen Einstellparameters im Element nach Sendung des Aktivierungssignals.

14. Verfahren nach Anspruch 13,
**dadurch gekennzeichnet, dass** vor dem Einstellen des Strahlpfades zur Festlegung des einzustellenden Strahlpfades ein Anforderungssignal von einem der Bestrahlungsplätze an die Zuordnungseinheit, insbesondere über eine direkte und fest zugeordnete Signalverbindung, gesendet und eine Verfügbarkeit des Partikelstrahls überprüft wird und dass bei einer Verfügbarkeit des Partikelstrahls dieser dem anfordernden Bestrahlungsplatz zugeteilt wird.

15. Verfahren nach Anspruch 14,
**dadurch gekennzeichnet, dass** nach dem Zuteilen zur Bestrahlung benötigte Parameter, insbesondere Parameter des angeforderten Partikelstrahls und des Strahlpfades, von einer Kontrolleinheit des den Partikelstrahl anfordernden Bestrahlungsplatzes an eine Beschleunigerkontrolleinheit zur Einstellung und Kontrolle von zur Bestrahlung benötigten Parametern der Beschleuniger- und Partikelstrahlzuführungseinheit übermittelt wird und dass anschließend von der Beschleunigerkontrolleinheit an das mindestens eine Element der mindestens eine Einstellparameter gesendet wird.

16. Verfahren nach Anspruch 14 oder 15,
**dadurch gekennzeichnet, dass** nach dem Zuteilen ein Bestätigungssignal an den anfordernden Bestrahlungsplatz über eine direkte und fest zugeordnete Signalverbindung oder über ein Datenbus-System, an das die Kontrolleinheit und die Zuordnungseinheit angebunden sind, geschickt wird.

17. Verfahren nach Anspruch 16,
**dadurch gekennzeichnet, dass** das Aktivierungssignal gesendet wird, nachdem das Bestätigungssignal bestätigt wurde.

18. Verfahren nach einem der Ansprüche 13 bis 17,
**dadurch gekennzeichnet, dass** nach Senden eines Bestätigungssignals an einen Bestrahlungsplatz, der kein Anforderungssignal gesendet hat, die Partikelbeschleunigung und/oder einer Weiterleitung der Partikel, insbesondere von der Zuordnungseinheit und/oder der Kontrolleinheit, unterbrochen wird.

19. Verwendung einer direkten und fest zugeordneten Signalverbindung zwischen einem zur Einstellung eines Strahlpfades in einer Partikeltherapieanlage einstellbares Element und einer Zuordnungseinheit einer Partikeltherapieanlage zur Übermittlung eines Aktivierungssignals.

20. Vorrichtung zur Einstellung eines Strahlpfades zu einem von mindestens zwei Bestrahlungsplätzen einer Partikeltherapieanlage, wobei der Strahlpfad in einer Beschleuniger- und Partikelstrahlzuführungseinheit der Partikeltherapieanlage mittels mindestens einem einstellbaren Element einstellbar ist und wobei die richtige Einstellung des Strahlpfades mittels einer Zuordnungseinheit zur Sicherstellung und Überwachung einer korrekten Partikelstrahlführung gewährleistet wird, aufweisend:
- Mittel zum Senden von mindestens einem Einstellparametern an das mindestens eine Element,
- Mittel zum Senden eines Aktivierungssignals über eine direkte und fest zugeordnete Signalverbindung von der Zuordnungseinheit an das mindestens eine Element,
- Mittel zum Umsetzen des mindestens einen Einstellparameter im mindestens einen Element nach Sendung des Aktivierungssignals.

21. Vorrichtung nach Anspruch 20,
**dadurch gekennzeichnet, dass** Mittel zum Empfang eines Anforderungssignals, Mittel zur Überprüfung einer Verfügbarkeit des Partikelstrahls und Mittel zur Zuteilung des Partikelstrahls an den anfordernden Bestrahlungsplatz vorhanden sind.

22. Vorrichtung nach Anspruch 20 oder 21,
**dadurch gekennzeichnet, dass** Mittel zur Übermittlung von zur Bestrahlung benötigten Parametern, insbesondere Parametern des angeforderten Partikelstrahls und des Strahlpfades, von einer Kontrolleinheit des den Partikelstrahl anfordernden Bestrahlungsplatzes an eine Beschleuni-Strahlung benötigten Parametern der Beschleuniger- und Partikelstrahlzuführungseinheit vorhanden sind.

23. Vorrichtung nach einem der Ansprüche 20 bis 21,
**dadurch gekennzeichnet, dass** Mittel zum Senden eines Bestätigungssignals an den Bestrahlungsplatz vorhanden sind, wobei das Bestätigungssignal insbesondere über eine weitere direkte und fest zugeordnete Signalverbindung oder über ein Datenbussystem, an das die Kontrolleinheit und die Zuordnungseinheit angebunden sind, sendbar ist.

24. Vorrichtung nach einem der Ansprüche 20 bis 23,
**dadurch gekennzeichnet, dass** Mittel zum Unterbrechen einer Partikelbeschleunigung und/oder einer Weiterleitung der Partikel, insbesondere angesteuert durch die Zuordnungseinheit und/oder die Kontrolleinheit, vorhanden sind.
